# EUROPEAN PATENT APPLICATION

(11) **EP 3 249 565 A2**
(43) Date of publication of application: **29.11.2017**
(21) Application number: 17182713.2
(22) Date of filing: 24.07.2017
(51) Int. Cl.: G06F 19/00, G06Q 50/22

(54) **AUTOMATED APPLICATION SELECTION FOR MEDICAL DEVICES**

(30) Priority: 29.07.2016 US 201615223274
(71) Applicant: Siemens Healthcare GmbH, 91052 Erlangen (DE)
(72) Inventor: Dominick, Lutz, 91330 Eggolsheim (DE); Ukis, Vladyslav, 90489 Nürnberg (DE)

(57) **Abstract**

Systems and methods are provided for automatic application selection for a medical imaging device. A processor receives scan data from a medical imaging device. The medical imaging device is identified using the scan data. One or more applications related to the medical imaging device are matched in an applications database. The processor arranges for integration of the one or more applications on a hospital network.

## Description

### TECHNICAL FIELD

The present embodiments relate to medical imaging devices and application selection.

### BACKGROUND

Hospitals, diagnostic centers and medical imaging centers may use multiple types of equipment. A diverse collection of imaging or scanning devices may be used to provide services to patients and/or assist in diagnosis. A single hospital may, for example, provide x-ray examinations, computed tomography (CT) imaging, ultrasound, magnetic resonance imaging (MRI), positron emission tomography (PET) imaging, and interventional radiology among other procedures or imaging techniques. Each procedure may involve one or multiple machines and imaging devices. Each machine or imaging device may require multiple formats and settings. The output of each machine or imaging device may require a specific application or process to view or evaluate the outputted data. The data generated by each machine may be stored in different formats or locations. In total, the technology requirements to maintain an updated and efficient hospital, medical imaging center, or diagnostic center is challenging. Each device may require a separately defined pathway from imaging to evaluation involving many different applications and formats. Further, each of the individual devices and applications that make up the pathways may be updated or altered requiring the pathway to constantly be reevaluated so the correct devices are communicating with the correct applications in the correct way.

As each new device is added to the collection and more information is acquired, the collection of devices and types of stored data grow in complexity. There is a technical challenge to create a bundle of dedicated applications that fit with the complexity and structure of collection of devices, fit with the technical format and semantic variation in the data history, and capable of presenting the scan data to a user.

### BRIEF SUMMARY

Embodiments are provided for automatic application selection for a collection of medical devices.

In one aspect, a system for automatic application selection is provided. The system includes an imaging device, a PACS, a user interface, an appstore, and a chainsmith module. The imaging device is configured to generate scan data. The PACS is configured to store the scan data. The user interface is configured to present the scan data and information related to the scan data. The appstore is configured to store one or more applications for each of the imaging device, the PACS and the user interface. The chainsmith module is configured to identify the imaging device from the scan data and identify an imaging device application in the appstore related to the imaging device.

In another embodiment, a method for automatic application selection for a medical imaging device. A processor receives scan data from a medical imaging device. The medical imaging device is identified using the scan data. One or more applications related to the medical imaging device are matched in an applications database. The applications are integrated into the hospital network and connected to the medical image device.

In another embodiment, an apparatus is provided for automatic application selection. The apparatus is configured to analyze the data of a plurality of medical imaging devices. One or more interpretation applications related to one or more medical imaging devices are identified in an applications database. The apparatus is configured to analyze a plurality of PACS. One or more query retrieve applications related to one or more PACS are identified in the applications database. The apparatus transmits a notification to the hospital network including the identification of the one or more interpretation applications and the one or more query retrieve applications.

The present invention is defined by the following claims, and nothing in this section should be taken as a limitation on those claims. Further aspects and advantages of the invention are discussed below in conjunction with the preferred embodiments and may be later claimed independently or in combination.

### BRIEF DESCRIPTION OF THE DRAWINGS

The components and the figures are not necessarily to scale; emphasis instead being placed upon illustrating the principles of the invention. Moreover, in the figures, like reference numerals designate corresponding parts throughout the different views.
Figure 1 depicts an example of communication pathways in a system.
Figure 2 depicts an example system for selecting a bundle of applications for a collection of devices.
Figure 3 depicts an example chainsmith module of the system of Figure 2.
Figure 4 depicts an example workflow for selecting a bundle of applications for a collection of medical imaging devices.
Figure 5 depicts an example system for selecting a bundle of applications for a plurality of collections of devices.

### DETAILED DESCRIPTION

Embodiments describe a system and method for selecting and generating a bundle of dedicated applications that fit within the complexity and structure of a medical imaging collection of devices.

A hospital may have a diverse collection of devices. For example, a hospital may use multiple different imaging devices to perform diagnostics. Even with similar devices or similar families of devices, each device may be operated differently due to differences in the device's vendor, the version of the device, or any update done to one but not another device. In addition, a hospital may contain diverse archival or storage systems or formats (e.g. picture archiving and communication systems or PACS). A hospital may have multiple different networking protocols. Finally, a hospital may use different types of interfaces or evaluation applications. The permutations generated by the different devices, storage, interfaces, and protocols generate a huge variety of data and technology requirements. Each of these permutations may use a unique application relative to other permutations, resulting in a variety of applications. The variety generates the possibility of multiple different pathways from input to output that may each involve separate applications to perform.

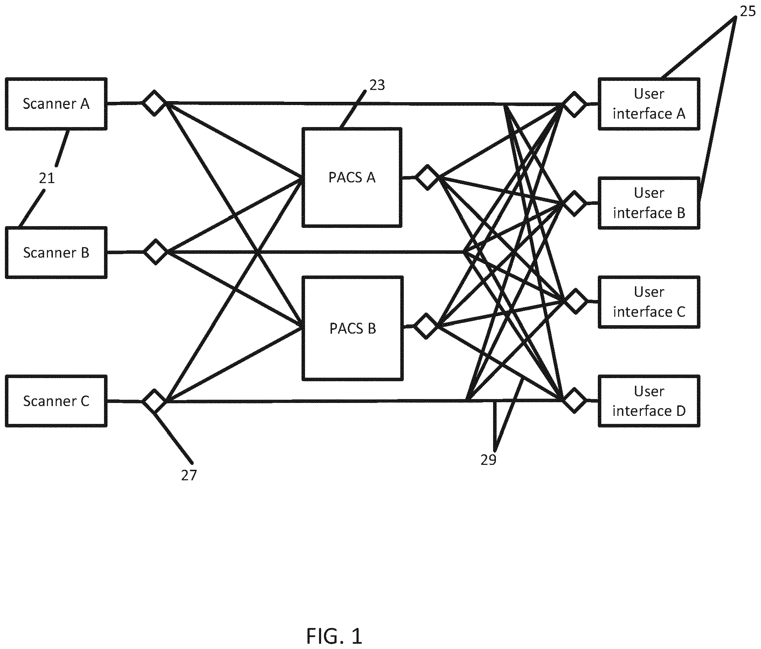

Figure 1 illustrates pathways 29 from scanning devices 21 to user interfaces 25. The hospital in Figure 1 uses three different scanners 21 and two PACS 23. There are four user interfaces 25. For the system in Figure 1, the scanners 21 (scanners A, B, and C) may be configured to capture scan data relating to an object (e.g. a portion of patient). The PACS 23 (PACS A and B) may be configured to store scan data. The user interfaces 25 (User Interfaces A, B, C, and D) may be configured to present information related to the scan data. Figure 1 also includes nodes 27 (or input / outputs) and pathways 29 between the inputs and outputs of each device 21, PACS 23, and user interface 25. Each pathway may represent the transmission of data from generation to evaluation. There are thirty-six possible pathways from scanner 21 to user interfaces 25. For example, data generated at scanner A may first go to either PACS A or B or directly to the user interfaces A, B, C, D. If the scan data is stored in the PACS 23, there are then four possible outputs from the PACS 23 to the user interfaces 25 for each of the PACS A and B. For each connection between devices 21, PACS 23, and user interfaces 25, there may exist an application to receive, interpret, retrieve or evaluate the scan data. Figure 1 illustrates a simple system with only a few devices 21, PACS 23 and user interfaces 25. Different numbers of scanners, PACS, user-interfaces, and/or pathways may be provided. As additional components are added, the complexity of the pathways and interconnections between the components grows larger.

Part of the combinatory complexity has been solved by the implementation of a universal format for storage and transfer. For example, the DICOM (Digital Imaging and Communications in Medicine) format creates a template for each device or component to work off of. However, DICOM is only an architecture on which systems and applications may be built. Each component of the chain from scan to evaluation may require the right data to be input and may require a specific application to read the output. A component that requires a specific field inputted may have an error if the field is not formatted or entered correctly. Each component may be programmed or setup to use specific information. In order to tie the components together to generate useful information at a user interface, even using DICOM, each component may work in concert to agree on the data transferred. Each component may output data in a first format that is read by a subsequent component. Even if the system is setup to function properly at first, any subsequent changes may alter the format of the outputted data and ability of the subsequent component to understand the data. For example, when a new use interface application is implemented (e.g. to evaluate the dose used to acquire the images), the PACS may be required to update to a new application that understands a new query retrieve procedure, that may further impact scanning devices and how the data from the scanning devices is interpreted by the PACS. For a simple system, the number of possible combinations may be low. However, as additional devices, manufactures, device types, modality types, optimization steps, versions, and/or updates enter the system, the number of combinations increases exponentially.

The disclosed embodiments may be implemented to automatically identify and connect the proper applications leading to an improvement in the computational system. Applications are automatically identified that allow for communication between different devices in order to improve the efficiency and function of a medical imaging system. The increased efficiency and usage of resources may lead to quicker turnaround times, fewer communication errors, and less maintenance for end users.

Embodiments provide systems and methods to handle the combinatory complexity for connecting devices and data in a collection of medical devices. A central control unit or chainsmith analyzes data from the devices and analyzes the possible applications stored in an applications database. Based on the analysis, the chainsmith correctly selects a bundle of applications and integrates the bundle of applications into a network containing the devices.

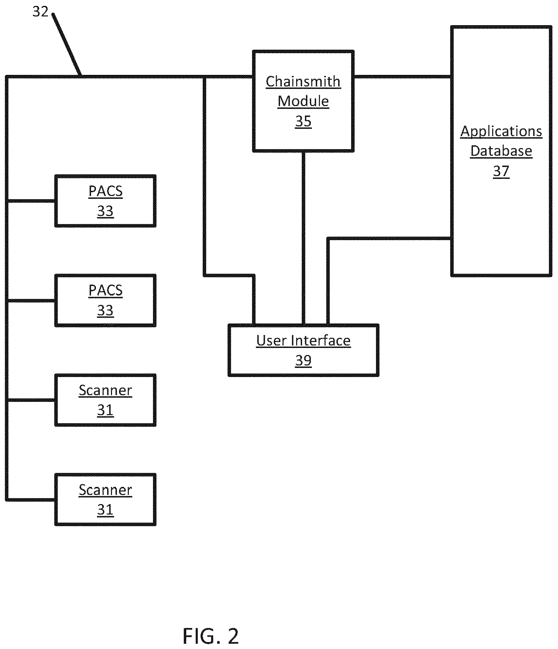

Figure 2 illustrates an example system for selecting a bundle of applications for a collection of medical devices. Figure 2 includes a plurality of scanning devices 31(scanners) and a plurality of PACS 33. The scanning devices 31 and PACS 33 communicate with a chainsmith module 35 over a network 32. The chainsmith module 35 communicates with an applications database 37 (appstore) over the network 32. The user interface 39 may communicate with the PACS 33 and scanners 31. Additional, different or fewer components may be included. For example, Figure 2 illustrates components of a hospital imaging center. In addition to or as an alternative, the system may include other components. The system is described as using imaging machines such as scanners 31 and PACS 33. The system may include other medical devices used as diagnostic tools such as laboratory testing devices, therapy devices, other networking devices, and/or other storage devices for example. Components of the system may be located on site at a hospital or in the cloud. In certain embodiments, the components may operate automatically without user input. In certain embodiments, the components may operate concurrently, or may operate as a result of an input from a user or an application.

The network 32 is a hospital, medical, or other local area or enterprise network with or without connection to or inclusion of a wide area network or the Internet. The network 32, through which the components may communicate, may include wired networks, wireless networks, or combinations thereof. The wireless network may be a cellular telephone network, LTE (Long-Term Evolution), 4G LTE, a wireless local area network, such as an 802.11, 802.16, 802.20, WiMax (Worldwide Interoperability for Microwave Access) network, or wireless short range network. Further, the network 32 may be a public network, such as the Internet, a private network, such as an intranet, or combinations thereof, and may utilize a variety of networking protocols now available or later developed including, but not limited to transmission control protocol/internet protocol (TCP/IP) based networking protocols.

The system of Figure 2 includes a plurality of scanning devices 31. Scanning devices may also be referred to as imaging devices or scanners. Such scanners 31 may include, for example, magnetic resonance imaging (MRI) scanners, positron emission tomography (PET) scanners, Single-photon emission computed tomography (SPECT) scanners, ultrasound scanners, tomography devices, among others. The scanning devices 31 may include any type of imaging device, medical diagnostic device, and/or therapy device that is connected to a medical or hospital network.

Each scanning devices 31 may be configured to interface with an application specific to the scanning device 31. Each scanning device 31, for example, may output a specific set of scan data. Different devices may integrate with different applications. For example, a MRI device may include or require a different interpretation application than a PET device. The MRI device may require different inputs or fields. The PET device may use different settings and require different inputs. Both devices may output different types or sets of data in a standard or different format. Different manufacturers may use different software and different formats for similar types of devices. Different versions of the same machine may output different formats. Even a similar version of a scanning device 31 from the same manufacturer may require a different interpretation application.

Each scanning device 31 may be configured to generate data (such as scan data) relating to a patient. The scanning devices 31 may output using a formatting standard such as DICOM standard for formatting the data. DICOM is a standard used for storage and transmission of medical scan data, such as two dimensional images, three dimensional images, and video images. The scanning devices 31 may generate data using the Health Level-7 (HL7) standard. Other formats or standards may be used by different devices. The standards may be hospital specific or vender specific. For example, a vendor may maintain a specific standard or format for each of the vendor's scanning devices 31 in order to maintain control over the data and require that each scanning device 31 conform that that vendor's standard.

The generated data from the scanning device 31 may include both scan data and protocol data related to the scan or study. The scan data may be formatted using standard image formats and may, for example, be a single image or a collection of images. The scanning devices 31 may generate one or more two-dimensional images that are subsequently stitched together to create a three-dimensional image or a moving image. The related protocol data may include information regarding the scan (e.g. the type of scan, scan settings, the region of the body, patient information). For example, when using the DICOM standard, the generated data may include a network message that is initiated by the scanning device. The message, referred to as a Modality Performed Procedure Step (MPPS), may also be sent to the Picture Archiving and Communication System (PACS). The message carries information about what was performed by the scanning device during acquisition. Other messages that include information describing the type and model of scanner may be sent from the scanning device 31 to the chainsmith module 35. Using DICOM, each scanning device 31 may use a similar format to output data. However, the outputted data may vary depending on the type of scanning device 31, manufacturer, modality, version, and/or expected use.

A hospital or imaging center may include multiple scanning devices 31. The scanning devices 31 may be updated or replaced as technology is implemented. Updates may include both hardware updates and software updates. Parts or components such as the user interface for a scanning device 31 may be altered over time. An interpretation application that is integrated with the scanning device 31 may require updates if the hardware is changed. Workflow changes may also require the application be updated. For example, if a hospital requires a different type of patient field or billing field be entered prior to starting the study, the application that interprets the output of the scanning device 31 may be updated. With each update, the scanning device 31 may communicate differently with the PACS 33; e.g. output different data or different types of data. Each change may therefore require a redefining of the relationship between the scanning device 31 and other devices.

The system in Figure 2 also includes one or more PACS 33. PACS 33 may be configured to transfer, store, display and/or manage medical data such as images and related information. PACS 33 may include multiple servers and multiple storage devices. Each PACS 33, for example, may include a processor and memory including multiple RAID arrays for storage of image or picture data. A PACS 33 may be located on site at, for example, a hospital or the PACS 33 may be located in the cloud using shared processing and resources. The PACS 33 may incorporate an internal network to transfer data from storage to workstations.

Each PACS 33 may be configured to store data (such as scan data). A PACS 33 may use a standard format such as DICOM or HL7. DICOM, for example, specifies the fields and types of data that may be stored in a PACS 33. The PACS 33 receives and stores data from the scanning devices 31. The PACS may receive requests from the user interface for information regarding a scan or scan data. The PACS may use an application to interpret requests from the user interface. The PACS 33 may store raw scan data or processed scan data. The PACS 33 may facilitate transmission of data from the scanning devices to a user interface. The PACS 33 may require an application to communicate with each scanning device. A scanner interpretation application may be used to convert the output of the scanning device 31 to a data format that is readable and storable in the PACS 33. The PACS may store information from multiple scanning devices in potential multiple formats. The formatting and storage of data may change over time as changes are made to the DICOM standard.

The system in Figure 2 includes one or more application databases 37 referred to as appstores 37. An appstore 37 may include one or multiple servers or computing devices. The appstore 37 may be a collection of multiple vendor specific servers. For example, the appstore 37 may include a first server that stores applications for vender A, a second server that stores applications for vender B and so on. In certain embodiments, the appstore 37 may include different servers for different types of applications. In certain embodiments, the appstore 37 is a single database containing application for multiple vendors. The appstore 37 may serve as a clearinghouse for applications for all medical devices. The appstore 37 may include one or more databases that contains a list of applications and attributes for the applications. The appstore 37 may be located on site (e.g. at the hospital) or in the cloud. The appstore 37 is connected to the chainsmith module 35 through a network, such as the network 32 or another network. The appstore 37 may further be connected to the scanning devices 31 and PACS 33 in order to update the applications associated the scanning devices 31 and PACS 33.

An appstore 37 may be configured to serve as a repository for one or more applications or software packages. The appstore 37 may include multiple types of applications that may be used in a hospital setting. Different types of applications may include applications for interpreting each individual scanning device, applications for the PACS, applications for evaluation of the scan data, and applications that coordinate communications between each of the devices. Interpretation (INT) applications for imaging device / scanner data interpretation may be stored in the appstore 37. Query retrieve (Q/R) applications used to query and read from vender specific PACS 33 may be stored in the appstore 37. MED applications data consolidation, interpretation, and merging may be stored in the appstore 37. User interface applications (e.g. business intelligence specific tasks) such as dose, usage, or evaluation applications may be stored in the appstore 37. Other types of applications may be stored in the appstore 37.

INT applications may be configured for scanning device 31 and scanner data interpretation. INT applications may be configured to input data from scanning devices 31 and output data in a format that is readable by a PACS, PACS applications, MED applications, and user interface applications. INT applications may provide translation of the data from the scanning devices 31 to the PACS 33, to a MED application, a user interface, or a user interface application.

Q/R applications may be configured to read data from the PACS 33. The PACS 33 may receive a request for internally standardized queries from, for example, a user interface or user interface application. The Q/R application transforms the request to a set of suitable queries, and returns the results.

MED applications may be configured for as services for data consolidation. Data consolidation might be used to merge data from the devices or INT applications. For example, DICOM Dose Information may be merged from multiple INT applications into one single input for the user interface of e.g. dose management application, in a way that shields the multitude of INT applications from the dose management application and makes the latter independent of both the variety of INT applications and the variety in the data that was transformed by the INT applications.

User interface applications may be configured for business intelligence specific tasks (e.g. dose, usage, protocol evaluations, etc.) and dedicated applications for services such as image processing and display. User interface applications may communicate with a scanning device or a MED application. For example, the scanning device may output data that is merged by the MED application that is then used in a user interface application. User interface applications may communicate with a PACS 33. User interface application may evaluate scan data for different criteria.

The applications in the appstore 37 may be maintained and updated by a respective vendor. For example, an application for a device A manufactured by company A may be updated by company A. Applications may be created and updated by third parties. For example, a company may create an application for a device that the company does not manufacture. In certain embodiments, a vendor may be responsible for updating each application. Applications may be created by multiple entities as interfaces between different devices of different vendors. For example, company A and company B may work together to make sure the scanner from company A works with the PACS 33 system of company B and the user interface of company C. The appstore may store multiple versions of an application. For example, even though an application has been updated to, for example, version 1.04, the appstore 37 may still maintain a copy of versions 1.03, 1.02, etc.

The applications in the appstore 37 may include metadata that relates to application. The metadata may include version information and version history. The metadata may include specific devices or interfaces with which the applications are verified to work. The appstore 37 may include other information relating to the scanning devices and the applications such as instructions for how to integrate or connect the application. The appstore 37 may include information such as what other applications are compatible with each application. The appstore 37 may include information relating to the format and inputs/outputs that each device and each application uses.

Referring back to Figure 2, the user interface 39 may include one or more workstations or terminals. The user interface 39 may be a mobile device such as a smartphone or tablet. The user interface 39 may be located in a reading room or similar environment. The user interface 39 may be accessible through the use of a network connection (wired or wireless) to a hospital. The user interface may be a virtual machine or accessible through a website. In one embodiment, the user interface 39 is a user interface for the appstore 37, PACS 33, chainsmith module 35, and/or scanner 31.

The user interface 39 may be configured to display, present, or output medical data. The user interface 39 may include a user interface application that allows a user to visualize the medical data generated by the scanning device 31. The user interface 39 may communicate directly with the scanning device 31 or may request information and data from a PACS 33. The user interface may be part of the PACS 33.

The user interface 39 may be configured to execute user interface applications that provide business intelligence. The user interface applications may input scan data and output processed scan data or annotated or evaluated scan data. The user interface applications may require a specific format for inputted data. Each user interface application may operate differently, using different inputted data, for different types of images or studies. User applications such as dose, usage, or protocol evaluations may be executed on the scan data from the scanning device or PACS 33. The user interface applications may be run in a workstation or server in for example, a reading room. The user interface applications may be executed in the cloud, at the scanning device, or at the PACS 33. The user interface applications may be selected to run by a technician or end user.

The system of Figure 2 includes a chainsmith module 35. The chainsmith module 35 may include one or more servers or computing devices. The chainsmith module 35 may be coupled with the one or more scanning devices 31 and the one or more PACS 33. The chainsmith may also be connected to the appstore 37. The chainsmith module 35 may include a communications module configured to communication over a network 32 with the scanning devices 31, PACS 33, and/or the appstore 37. The chainsmith module 35 may be located at the hospital or in the cloud. The chainsmith module 35 may be integrated into the appstore.

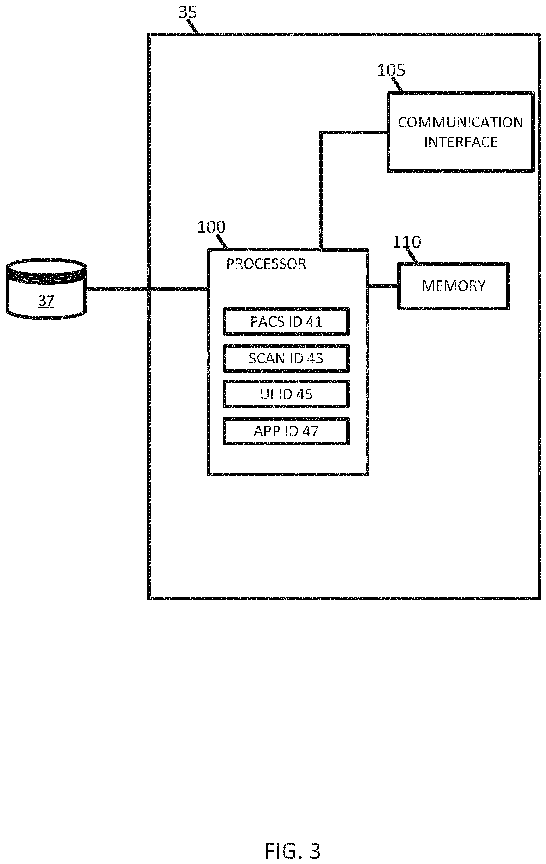

The chainsmith module 35 is configured to analyze the devices to determine a bundle of applications on the appstore 37 that efficiently connect the devices. Figure 3 illustrates an example embodiment of a chainsmith module 35. The chainsmith module 35 includes a processer 100, a memory 110, and a communications interface 105. The processor 100 may include a PACS identification module 41, a scanner identification module 43, a user interface identification module 45, and an applications identification module 47.

The processor 100 may include a general processor, digital signal processor, an application specific integrated circuit (ASIC), field programmable gate array (FPGA), analog circuit, digital circuit, combinations thereof, or other now known or later developed processor. The processor 100 may be a single device or combinations of devices, such as associated with a network, distributed processing, or cloud computing.

The memory 110 may be a volatile memory or a non-volatile memory. The memory 110 may include one or more of a read only memory (ROM), random access memory (RAM), a flash memory, an electronic erasable program read only memory (EEPROM), or other type of memory.

The communication interface 105 may include any operable connection. An operable connection may be one in which signals, physical communications, and/or logical communications may be sent and/or received. An operable connection may include a physical interface, an electrical interface, and/or a data interface. The communication interface 105 provides for wireless and/or wired communications in any now known or later developed format. In one embodiment, the communication interface 105 is a network interface card or cards.

The chainsmith module 35 may be configured to analyze the incoming data from the hospital site, in terms of PACS and scanner specifics, either based on medical standards (e.g. DICOM, HL7) and/or using an identification module.

The scanner identification module 43 may receive data generated from the scanner device. The data may be formatted using a medical standard such as DICOM. The DICOM information may include data that allows the scanner identification module 43 to identify the scanner and any protocol used for the scan or the study. The DICOM information may, for example, list the type of scan, the type of modality, and the outputted data (in the DICOM designated fields). In other embodiments, the scanner identification module 43 may identify the scanner without any specific structural or semantical pre-information. The scanner identification module 43 may convert the received scanner data or scanner information into a unified data format that indicates which scanner device produced the data, and how the data is encoded.

In certain embodiments, the scanner identification module 43 may search or query the hospital network to identify new machines or devices. For example, the scanner identification module may request information from each device connected to the network. Scanning devices may be queried to determine the type of device and version. In certain embodiments, new devices may transmit messages to the scanner identification module to identify the devices.

The PACS identification module 41 may be configured to identify the type and formatting of the PACS. The PACS identification module 41 may read PACS identification meta data from the stored DICOM data relating to the scan data. Other sources of the identification may be provided, such as pushing by the PACS to the PACS identification module 41 or accessing a database of identifications. The PACS identification module 41 may provide an internally standardized meta data format as output.

Using the information from scan data and other inputs, the scanner identification module may generate an inventory or hospital site layout of a collection of medical devices at a site (hospital). The chainsmith module 35 may store metadata relating to each device along with the integrated applications in a database. The chainsmith module 35 may attempt to determine connections for every device in the collection of devices, such as the communications paths of Figure 1. The database may be updated as the devices or application change over time.

The user interface module 45 may identify user interface applications used in the hospital. The user interface module 45 may monitor the workstations used by physicians or users to detect which applications are being used to evaluate the data. The user interface module may collect and store data regarding the inputs used by each of the user interface applications.

The chainsmith module 35 may be configured to analyze the current appstore 37 contents. The applications on the appstore may be compared to data and current status/layout of the collection of medical devices, based on meta information provided with each application in the appstore. The applications identification module 47 may be configured to communicate with the appstore 37. The application identification module 47 may be configured to receive scanner device and PACS identification information from the PACS identification module 41 and scanner identification module 43. The application identification module 47 may further be configured to receive information regarding a site layout of the devices and PACS. For example, the applications identification may use the connectivity of each device to search the appstore 37. Each application in the appstore may include information related to which devices the application is compatible with. The user interface applications used by the hospital (in for example, a reading room) may be compared with both alternative applications on the appstore.

The chainsmith module 35 may be configured to automatically select applications from the appstore that fit with the scanner data and PACS formatting. The scanner and PACS identification modules may identify the collection of devices in a hospital. The application identification module 47 may identify the application available in the application store that match the identified fleet of devices. INT applications may be selected that operate with each of the scanning devices. Q/R applications may be selected that operate with the identified PACS or storage devices. MED applications may be selected that operate with the medical network and formatting. User interface applications may be selected that operate with both the selected INT applications, Q/R applications, and correspond to user preferences and potential uses of the scanning data. For example, a MRI image viewing application may be selected that operates with both an INT application for the MRI device and a Q/R application for the PACS.

The chainsmith module 35 may download (or transmit a notification to the devices) the correct applications and make the application available to the devices of the collection. In an embodiment, the chainsmith module 35 is further configured to identify error or mismatches between the devices and applications. The chainsmith module 35 may be configured to correct the error or to send a notification to a responsible user. The chainsmith module 35 may have permissions to automatically download and integrate applications in the hospital network. In certain embodiments, the chainsmith module 35 may require an input from a user to accept any changes to the system. For example, an application may require a payment or subscription that may require authorization from a user. In certain embodiments, as the fleet of devices changes, the chainsmith module 35 may be configured to automatically update the customer/site specific fleet/layout.

In certain embodiments, the chainsmith module 35 is configured to automatically assemble a pathway or connection from a scanner 31 to the user interface 39. When a new scanner is detected and identified, the chainsmith module 35 may select an INT application to interpret the data from the scanner 31. The chainsmith module 35 may download and connect the scanner 31 to the INT application. The chainsmith module 35 may identify and verify that a Q/R application for the PACS 33 works with the INT application and the scanner 31. The PACS 33 may then be connected to the scanner 31 and the user interface 39. If there are any new user interface applications, the new user interface applications may be connected to both the PACS 33 and scanner 31. With any changes or updates, the chainsmith module 35 may continuously assemble a working and efficient pathway from scanning device 31 to user interface 39.

In certain embodiments, the chainsmith module 35 may receive feedback from the scanning devices 31, PACS 33, or user interface 39 requesting updates or identifying errors or issues. The chainsmith module 35 may be configured to select an alternative application in the appstore 37 if the existing application (while potentially correct from a technical standpoint) is causing errors.

In certain embodiments, the chainsmith module 35 may be configured to score or rank applications based on the feedback received from the scanning devices 31, PACS 33, user interface 39, or end users. The applications may be ranked for quality and then selected based on the ranking. In certain embodiments, the chainsmith module 35 may be configured to select a vendor approved application over a third party application.

In certain embodiments, the chainsmith module 35 may be configured to alert a vendor when an application is missing or mismatched on the appstore. The chainsmith module 35 may identify a missing application when querying the appstore 37. The chainsmith module 35 may, for example, identify a scanner 31 in the collection as scanner A version 1.10. However, the appstore 37 may only contain an application that is capable of interpreting the data from scanner A version 1.08. The chainsmith module 35 may then send a notification to both an administrator of the collection, an administrator of the appstore, and a developer of application (or the vendor of scanning device).

Each device in the fleet of devices may be configured to transfer data and communicate with other devices. For example, a scanner 31 may communicate with a PACS 33 that may communicate with a user interface 39. A common language may be used to translate between devices. The data from each device may be translated into the common language. The output of each device may be interpreted so that the next device in the pathway may be able to perform its duty.

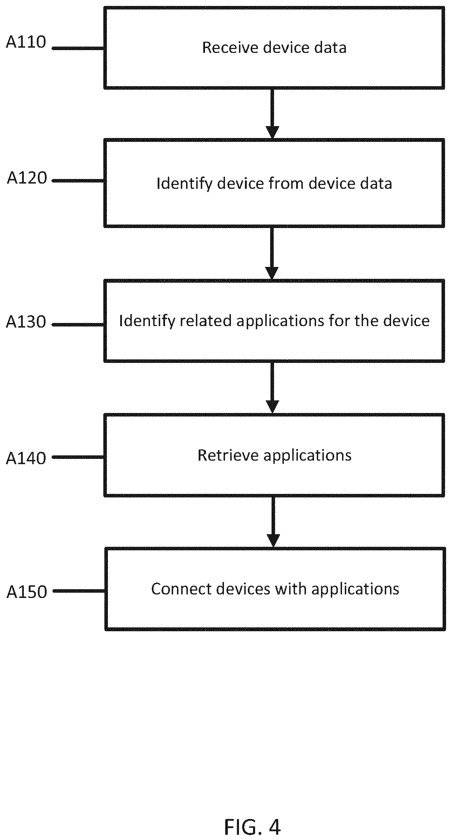

Figure 4 illustrates an example workflow for selecting a bundle of application for a fleet of medical imaging devices. In one embodiment, the acts are performed by the chainsmith module 35, such as a chainsmith server. Additional, different, or fewer acts may be provided. The acts are performed in the order shown or other orders. The acts may also be repeated.

At act A110, device data is received from a medical imaging device. Device data may include results data such as scan data or diagnostic data. The results data may be formatted using a medical standard such as DICOM or HL7. A medical data standard may include a file format definition and may further define transmissions protocols. Device data may include data related to a study done by the medical imaging device. A DICOM formatted file may contain additional information related to the study in a preamble or a header. Information related to the version and syntax of the formatted data load may also be included.

The device data is received directly from the scanner or device. In certain embodiments, the device data is received from a PACS. A scan or study may have previously occurred. The data from the scan or study may be stored in the PACS system until requested by a user or another process.

The device data may include information related to the destination of the data. For example, data from a MRI device may indicate that it will be stored in a type of PACS system and eventually viewed by certain types of user interface applications.

At act A120, the medical imaging device is identified using the device data. The device data may contain an explicit reference to the make and model of the device. For example, a serial number of the machine may be embedded in the device data. The serial number may be stored in a database on the server. The device data may contain information relating to the version or configuration of the imaging device. For example, the device data may include information that indicates that the imaging device uses a certain version of DICOM to communicate.

In certain embodiments, the identity of the medical imaging device may be determined by the format of the device data. The device data may only contain information for certain fields. The server may be able to identify the type (and vendor or model number) of the imaging device by comparing the type and amount of data outputted by the medical imaging device against a stored list of imaging devices and the respective outputted data. The outputted data may function as a signature for the respective imaging device. For example, a MRI device from vendor A may output a different set of fields than a MRI device from vendor B. Similarly, a version 1.02 of an MRI device may output a different set of data than version 1.04.

In certain embodiments, the device data may be received from a PACS. The identity of the PACS may be determined by the format of the device data. The format of the data may be used as the identifier as opposed to the identity of the PACS. The PACS may alternatively be identified by actively requesting identification.

At act A130, one or more applications related to the medical imaging device are identified in an applications database. The applications database may be a repository for multiple applications. In certain embodiments, the applications database may be a list with instructions on how to procure or download applications from, for example, a vendor site. The applications database may store metadata related to each application and a list of medical imaging devices and associated metadata. The server may query the applications database for a matching application for a different application or a medical imaging device.

The one or more applications may include applications for operating or interfacing with the imaging devices or PACS. The one or more applications may include applications for facilitating transfer of data from an imaging device to the PACS or to a user interface (or user interface application). The applications may interpret data from the imaging device and transform the data or augment the data so that the data may be read or stored. The applications may read the data from a PACS device and transform or augment the data so that the data may be used (processed, displayed, evaluated) by a user interface application.

Q/R Applications may be used to read from PACS that receive a request for an internally standardized query. The Q/R applications may transform the request to a set of suitable queries and return the results. Interpretation applications may be used for medical device/scanner data interpretation. Medical standard related applications may be configured for use as services for data consolidation (DICOM, HL7, etc.). User interface applications may be configured for business intelligence specific tasks (Dose, Usage, Protocol evaluations, etc.) and dedicated applications for user services.

Applications may be submitted to the appstore by developers or venders. The appstore may request specific applications from developers or venders where there is a need.

At act A140, the one or more applications are retrieved using the applications database. The applications may be connected and integrated into a hospital network. The applications may be downloaded from the applications database or from another location identified by the applications database. In certain embodiments, if there is not an application that meets the requirements for the medical imaging device, the server may send a notification to the developer community.

At act A150, the medical imaging device and the applications are connected. The server may integrate or install the application at the site. The applications may reside on a server or network in the hospital. The application may be installed at one or more networking devices or servers. The application may be stored in the cloud and run on demand when data is transmitted from an imaging device or from a PACS. In certain embodiments, the server may update the applications if the medical imaging device or the applications change.

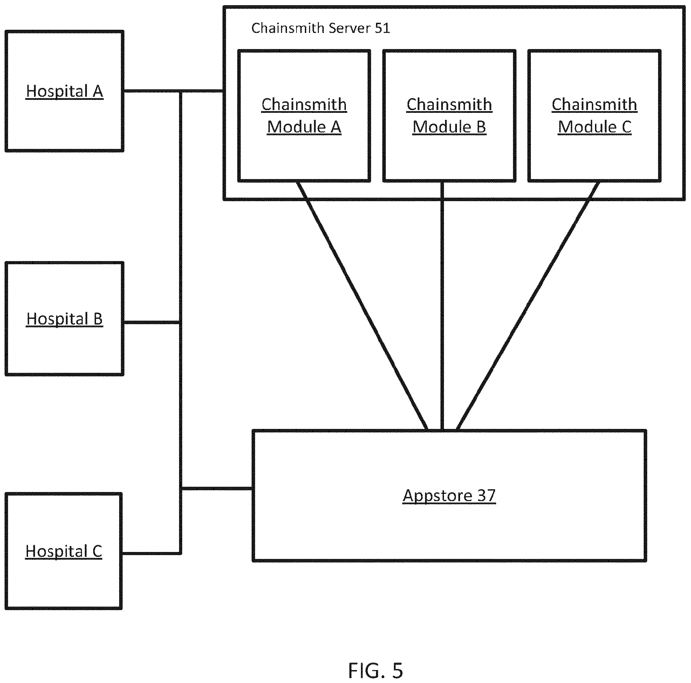

Figure 5 illustrates an example system for selecting a bundle of applications for a plurality of collections of devices. In certain embodiments, the applications database 37 or appstore may be used by a plurality of sites to maintain the collection of devices at each site. Figure 5 illustrates three different hospitals that communicate with the server 51. In this embodiment, the server 51 may be located in the cloud and connect to the hospitals over the network.

The Hospitals A, B, and C receive data from medical devices (such as imaging devices and scanners) and storage devices (such as PACS) and communicate with the server 51. The hospitals A, B, and C may maintain an inventory or layout of the respective imaging devices. In certain embodiments, the chainsmith modules A, B, and C store a copy of the inventory.

The chainsmith server 51 may include one or more chainsmith modules (A, B, and C) that are responsible for maintaining the applications for each fleet of devices. For example, chainsmith module A may be responsible for the fleet of devices at hospital A; chainsmith module B may be responsible for the fleet of devices at hospital B; and so on.

The chainsmith server 51 may be connected to the appstore 37 through the network. In certain embodiments, the server 51 is co-located with the appstore 37. In other embodiments, both the server 51 and appstore 37 may be located in the cloud.

The appstore 37 may contain one or more applications that relate to medical data. The application may include one or more non-user facing application such as Q/R implementation applications and scanner data interpretation applications. Q/R implementation applications may be used to access a PACS. Scanner data interpretation applications may be used to interpret data from scanning devices. User interface applications may be used to evaluate data at a user interface.

Each chainsmith module A, B, and C may operate independently for each hospital A, B, and C. In certain embodiments, each chainsmith modules is located on site at each of the hospitals. The appstore 37 may be located in the cloud and accessible to each hospital over the network. The appstore 37 may be used as a singular point of maintenance so that only one copy of an application is updated instead of having to update each application at each site. Any changes to an application may be pushed out to each hospital from a single site in the cloud.

The chainsmith modules may send the applications to the hospital network or send a notification indicating that the applications should be connected or integrated. The notification may list the devices and the associated applications. The hospital network may automatically connect the applications. The notification may be sent to the individual devices. In certain embodiments, the chainsmith modules transmits and integrates the applications into the hospital network. The chainsmith modules may integrate or connect the applications so that the imaging devices, PACS, user interfaces, and other devices are connected and capable of communicating with one another.

The illustrations of the embodiments described herein are intended to provide a general understanding of the structure of the various embodiments. The illustrations are not intended to serve as a complete description of all of the elements and features of apparatus and systems that utilize the structures or methods described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

While this specification contains many specifics, these should not be construed as limitations on the scope of the invention or of what may be claimed, but rather as descriptions of features specific to particular embodiments of the invention. Certain features that are described in this specification in the context of separate embodiments may also be implemented in combination in a single embodiment. Conversely, various features that are described in the context of a single embodiment may also be implemented in multiple embodiments separately or in any suitable sub-combination. Moreover, although features may be described above as acting in certain combinations and even initially claimed as such, one or more features from a claimed combination may in some cases be excised from the combination, and the claimed combination may be directed to a sub-combination or variation of a sub-combination.

Similarly, while operations are depicted in the drawings and described herein in a particular order, this should not be understood as requiring that such operations be performed in the particular order shown or in sequential order, or that all illustrated operations be performed, to achieve desirable results. In certain circumstances, multitasking may be advantageous. Moreover, the separation of various system components in the embodiments described above should not be understood as requiring such separation in all embodiments

One or more embodiments of the disclosure may be referred to herein, individually and/or collectively, by the term "invention" merely for convenience and without intending to voluntarily limit the scope of this application to any particular invention or inventive concept. Moreover, although specific embodiments have been illustrated and described herein, it should be appreciated that any subsequent arrangement designed to achieve the same or similar purpose may be substituted for the specific embodiments shown. This disclosure is intended to cover any and all subsequent adaptations or variations of various embodiments. Combinations of the above embodiments, and other embodiments not specifically described herein, will be apparent to those of skill in the art upon reviewing the description.

The Abstract of the Disclosure is provided to comply with 37 C.F.R. §1.72(b) and is submitted with the understanding that it will not be used to interpret or limit the scope or meaning of the claims. In addition, in the foregoing detailed description, various features may be grouped together or described in a single embodiment for the purpose of streamlining the disclosure. This disclosure is not to be interpreted as reflecting an intention that the claimed embodiments require more features than are expressly recited in each claim. Rather, as the following claims reflect, inventive subject matter may be directed to less than all of the features of any of the disclosed embodiments. Thus, the following claims are incorporated into the detailed description provided herein, with each claim standing on its own as defining separately claimed subject matter.

It is therefore intended that the foregoing detailed description be regarded as illustrative rather than limiting, and that it be understood that it is the following claims, including all equivalents, that are intended to define the spirit and scope of this invention.

## Claims

1. A medical imaging system comprising:
an imaging device configured to generate imaging device data;
a PACS in communication with the imaging device, the PACS configured to store the imaging device data;
a user interface in communication with the PACS, the user interface configured to present the imaging device data;
an appstore configured to store one or more applications for each of the imaging device, the PACS, and the user interface; and
a chainsmith module in communication with the imaging device, PACS, and user interface, the chainsmith module configured to identify the imaging device from the imaging device data, identify an imaging device application in the appstore related to the imaging device, and connect the imaging device to the imaging device application.

2. The medical imaging system of claim 1, wherein the chainsmith module is further configured to identify the PACS, identify a PACS application in the appstore related to the PACS, and connect the PACS to the PACS application.

3. The medical imaging system of claim 2, wherein the chainsmith module is further configured to identify a user interface application in the appstore and connect the user interface application to the PACS.

4. The medical imaging system of claim 1, wherein the imaging device is an ultrasound scanner, a magnetic resonance scanner, a computed tomography scanner, an x-ray scanner, a positron emission tomography scanner, or a single photon emission computed tomography scanner.

5. The medical imaging system of claim 1, wherein the imaging device data uses a DICOM standard.

6. The medical imaging system of claim 1, wherein the imaging device data uses a HL7 standard.

7. The medical imaging system of claim 1, wherein the imaging device application is configured to interpret the imaging device data.

8. The medical imaging system of claim 2, wherein the PACS application is configured to transform a request from the user interface into one or more queries of the PACS.

9. A method comprising:
receiving, by a processor, scan data from a medical imaging device;
identifying, by the processor, the medical imaging device using the scan data;
matching, in an application database, one or more applications related to the medical imaging device; and
arranging, by the processor, for integration of the one or more applications on a hospital network.

10. The method of claim 9 wherein the medical imaging device is an ultrasound scanner, a magnetic resonance scanner, a computed tomography scanner, an x-ray scanner, a positron emission tomography scanner, or a single photon emission computed tomography scanner

11. The method of claim 9 wherein identifying comprises:
determining a medical imaging device type, a vendor, and a model using DICOM data from the scan data.

12. The method of claim 11 wherein matching comprises:
matching the medical imaging device type, the vendor, and the model to one or more applications that have the same medical imaging device type, the same vendor, and the same model.

13. The method of claim 9 wherein the one or more applications are configured to interpret scan data from the medical imaging device.

14. The method of claim 9 wherein the medical imaging device is a PACS and the one or more applications are configured to query and retrieve scan data from the PACS.

15. The method of claim 9, further comprising:
updating the one or more applications related to the medical imaging device; and
connecting the medical imaging device and the one or more updated applications.

16. An apparatus comprising:
at least one processor; and
at least one memory including computer program code for one or more programs; the at least one memory and the computer program code configured to, with the at least one processor, cause the apparatus to at least perform:
analyze a plurality of medical imaging devices of a hospital network;
identify, automatically, one or more interpretation applications related to one or more medical imaging devices in the plurality of medical imaging devices in an applications database;
analyze a plurality of PACS in the hospital network;
identify, automatically, one or more query retrieve applications related to one or more PACS in the plurality of PACS in the applications database; and
transmit a notification to the hospital network including the identification of the one or more interpretation applications and the one or more query retrieve applications.

17. The apparatus of claim 16, wherein the at least one memory and computer program code are configured to cause the apparatus to further perform:
store a site layout identifying the plurality of medical imaging devices and the plurality of PACS in the hospital network.

18. The apparatus of claim 17, wherein the at least one memory and computer program code are configured to cause the apparatus to further perform:
detect a new medical imaging device in the plurality of medical imaging devices;
analyze the new medical imaging device; and
update the site layout describing the plurality of medical imaging devices and the plurality of PACS.

19. The apparatus of claim 16, wherein analyzing the plurality of medical imaging devices comprises:
identifying the one or more of the medical imaging devices in the plurality of medical imaging devices based on scan data received from the one or more medical imaging devices.

20. The apparatus of claim 16, wherein the at least one memory and computer program code are configured to cause the apparatus to further perform:
connect the one or more interpretation applications with the one or more medical imaging devices; and
connect the one or more query retrieve applications with the one or more PACS.
